Europäisches Patentamt

European Patent Office

Office européen des brevets

(11) Publication number: **0 327 648 B1**

(12) **EUROPEAN PATENT SPECIFICATION**

(45) Date of publication of patent specification: **09.06.93**

(51) Int. Cl.⁵: **C07K 15/04**, C12N 5/00, C12N 15/00, C12P 21/00, A61K 39/104, G01N 33/569, G01N 33/577

(21) Application number: **88900105.3**

(22) Date of filing: **14.12.87**

(86) International application number:
**PCT/JP87/00976**

(87) International publication number:
**WO 88/04669 (30.06.88 88/14)**

(54) **HUMAN MONOCLONAL ANTIBODY AND DRUG FOR PROPHYLAXIS AND TREATMENT OF INFECTIOUS DISEASES COMPRISING SAME AS EFFECTIVE INGREDIENT.**

(30) Priority: **15.12.86 JP 296594/86**

(43) Date of publication of application:
**16.08.89 Bulletin 89/33**

(45) Publication of the grant of the patent:
**09.06.93 Bulletin 93/23**

(84) Designated Contracting States:
**CH DE FR GB IT LI NL SE**

(56) References cited:
EP-A- 0 217 527     EP-A- 0 256 173
JP-A- 5 929 622     JP-A- 6 169 796
JP-A- 6 191 134     JP-A- 6 283 899
JP-A-61 155 398     JP-A-62 187 417

JOURNAL OF INFECTIOUS DISEASES, vol. 152, no. 6, December 1985, pages 1290-1299, The University of Chicago; S. SAWADA et al.: "A new common polysaccharide antigen of strains of Pseudomonas aeruginosa detected with a monoclonal antibody"

(73) Proprietor: **MITSUI TOATSU CHEMICALS, Inc.**
**2-5 Kasumigaseki 3-chome**
**Chiyoda-Ku Tokyo 100(JP)**

(72) Inventor: **FUKUDA, Tamotsu**
**2142, Togo**
**Mobara-shi, Chiba-ken 297(JP)**
Inventor: **ONO, Yasushi**
**13-4, Honcho 2-chome**
**Shiki-shi, Saitama-ken 353(JP)**
Inventor: **SHIGETA, Shiro**
**147-28, Omori-aza-kubouchi**
**Fukushima-shi, Fukushima-ken 960-11(JP)**
Inventor: **KUROIWA, Yasuyuki**
**2100, Togo**
**Mobara-shi, Chiba-ken 297(JP)**
Inventor: **OOKA, Hisayoshi**
**2142, Togo**
**Mobara-shi, Chiba-ken 297(JP)**

Rank Xerox (UK) Business Services
(3.10/3.6/3.3.1)

**ANTIBIOT. CHEMOTHER., vol. 36, 1985, pages
134-146, Karger, Basel, CH; J.C. SADOFF et
al.: "Characterization of mouse monoclonal
antibodies directed against Pseudomonas
aeruginosa lipopolysaccharides"**

(74) Representative: **Thomsen, Dieter, Dr.
Postbox 70 19 29
W-8000 München 70 (DE)**

## Description

The present invention relates to cells which produce human monoclonal antibodies to Pseudomonas aeruginosa each of which is single monoclonal antibody but shows affinity to 0-antigens of plural Pseudomonas aeruginosa of different serotypes, and said antibodies, and prophylactic and therapeutic agents against Pseudomonas aeruginosa infectious diseases containing the same as an effective ingredient as well as preparations thereof.

Pseudomonas aeruginosa is widely present in the natural world and can be found in drainages and in addition, the oral cavities or intestines of man and animals in a high frequency. This bacterium exhibits its pathogenicity in patients with general infectious diseases caused by virulence of this bacterium per se but rather in patients with reduced resistance to infection, namely, a cancer patient, a patient under immunosuppressive therapy, a transplant recipient, a patient with burn, a neonate and the like.

Pseudomonas aeruginosa infectious diseases would be infectious diseases currently considered to be most difficult for treatment. That is, Pseudomonas aeruginosa not only shows resistance to almost all antibiotics conventionally used hitherto but also tends to readily induce resistance to antibiotics developed in recent years. Taking the limit of antibiotic therapy into account, therefore, prophylaxis and therapy have been investigated, aiming at enhancing an ability capable of treating Pseudomonas aeruginosa in host. One is investigation on development of vaccine but it is difficult to expect an immediate effect on the patient who has already been infected. On the other hand, preparations comprising human immunoglobulin purified from healthy donors' sera or plasma, and its chemically modified product as an effective ingredient have been often used for treatment of Pseudomonas aeruginosa infectious diseases in recent years. Among antibodies contained in these preparations, however, an amount of an antibody having affinity to Pseudomonas aeruginosa and effective for the treatment is not constant and its amount is small so that many investigators cast a doubt on the prophylactic and therapeutic effect of these preparations. For this reason, development of human monoclonal antibodies effective in a small dose has been urgently desired.

On the other hand, as surface antigens of Pseudomonas aeruginosa, it is known that outer membrane protein (0MP), polysaccharide antigen derived from flagellum or slime and lipopolysaccharide (hereafter simply referred to as LPS) antigen are present. Among them, LPS is composed of 0-polysaccharide (hereafter sometimes referred to as 0-antigen) that is a serotype specific epitope, and lipid A and a core region which are a basic structure common to gram negative bacteria. 0-specific polysaccharide chain is composed of repetition of oligosaccharides. Serological classification of Pseudomonas aeruginosa is made by immunological property of this O-specific polysaccharide chain. However, even now, there is much argument about serological classification of Pseudomonas aeruginosa. In Japan, serological classification of the Serotyping Committee for the Japan Pseudomonas aeruginosa Society in which Pseudomonas aeruginosa is classified into 13 kinds from group A to group M has been widely used [Homma, Japan J. Exp. Med., 46, 329 - 336 (1976)]. The serological classification of the Serotyping Committee for the Japan Pseudomonas aeruginosa Society is compared with other classification, as described below.

3

| Comparison of serological classification of the Serotyping Committee for the Japan Pseudomonas aeruginosa society with other classification | | | |
|---|---|---|---|
| Serotyping Committee for the Japan Pseudomonas aeruginosa Society | Homma | Lanyi | Fisher |
| A | 1 | 1 | - |
| B | 2<br>7<br>13<br>16 | 3 | 3<br>7 |
| C | 3 | 5 | 6 |
| D | 4 | 10 | - |
| E | 5 | 7 | 2 |
| F | 6 | 11 | - |
| G | 8 | 4 | 1 |
| H | 9 | 2 | 5 |
| I | 10 | 6 | 4 |
| J | 11 | 12 | - |
| K | 12 | - | - |
| L | 14 | 13 | - |
| M | 15<br>17 | 9 | - |
| | - | 8 | - |

In recent years, a primary structure of O-specific polysaccharide chain has been clarified in proper course [for example, Kropinski et al., Antibiot. Chemother. 36, pgs. 58 - 73 (1985)] and there is a possibility that a new classification may be adopted in the future. It is known that an antibody to O-specific polysaccharide chain of LPS of Pseudomonas aeruginosa, namely, an antibody to Pseudomonas aeruginosa O-antigen, has a potent bacteriolytic or opsonic activity via the complement system. Aiming at obtaining human monoclonal antibodies effective for prophylaxis and therapy of Pseudomonas aeruginosa infectious diseases, it has been shown that human monoclonal antibodies to serotype antigens of Pseudomonas aeruginosa, namely, monoclonal antibodies capable of serotype specifically reacting with Pseudomonas aeruginosa, possess a very high protective activity against Pseudomonas aeruginosa infection of the same serotype; see JP-A-248626/1985. However, as is described in JP-A-152280/1986 and JP-A-155398/1986 which were subsequently published and relate to protection of human monoclonal antibodies to O-specific polysaccharide chains of LPS of Pseudomonas aeruginosa from infection, serotype specific human monoclonal antibodies reacting with Pseudomonas aeruginosa specifically react only with Pseudomonas aeruginosa of single serotype and have a protective activity against Pseudomonas aeruginosa infection of the same serotype but has no protective activity against Pseudomonas aeruginosa infection of other serotypes.

On the other hand, monoclonal antibodies to outer membrane protein of Pseudomonas aeruginosa [Sawada et al., J. Infect. Dis., 150, 570 - 576 (1985), Yoshiaki NAKAMURA et al., Jpn. J. Bacteriol., 39, 337 (1984)] or human monoclonal antibodies to common polysaccharides of Pseudomonas aeruginosa [Sawada et al., J. Infect. Dis., 150, 1290 - 1299 (1985)] are all low in their protective activity against Pseudomonas aeruginosa infection.

In Antibiot. Chemother. 36, pgs. 134-146 (1985) Sadoff et al. report about "Characterization of Mouse Monoclonal Antibodies Directed against *Pseudomonas aeruginosa* Lipopolysaccharides". Mouse monoclonal antibodies that react with O-side chain specific, O-side chain cross-reactive, and core P. *aeruginosa* lipopolysaccharide determinants have been isolated. The monoclonals directed at O-side chain determinants are generally opsonophagocytic with human neutrophils and human complement. They also protect mice from intraperitoneal and intravenous challenge and protect in the burned rat model of infection. In detail, the mouse monoclonal antibody 19.22.1 is referred to which reacts with Fisher serotypes 3, 7, 6, 5, and 2 (corresponding to groups B, C, H, and E according to the serological classification of the Serotyping Committee for the Japan Pseudomonas aeruginosa Society) and mouse monoclonal antibody 2.67.9.8 reacting with Fisher Serotypes 3, 5, and 2 (corresponding to groups B, H, and E).

Furthermore EP-256 713-A2 discloses human lymphoblastoid cell lines and hybridomas which secrete antibodies specifically reactive with lipopolysaccharide that is present on the surface of Pseudomonas aeruginosa. The individual monoclonal antibodies are immunospecific for one or more of the Fisher immunotypes of P. aeruginosa. The anti-Pseudomonas monoclonal antibodies are used individually or in combination to therapeutically or prophylactically treat P. aeruginosa infections. One or more of the monoclonal antibodies are co-administered with one or more antibiotics to therapeutically or prophylactically treat immunocompromised and immunosuppressed individuals. In detail, there is disclosed the anti-Pseudomonas aeruginosa human monoclonal antibody MAb-9H10, obtained from the EB virus-transformed cell line CF-9H10. It reacts only with lipopolysaccharide from Ps. aeruginosa Fisher immunotypes 3, 6, and 7; this means that it reacts with groups B and C of the classification of the Serotyping Committee for the Japan Ps. aeruginosa Society. The cell line CF-9H10 has been fused with immortalizing cells (KR4), and the most active hybridoma secreting MAb reactive with Ps. aeruginosa Fisher immunotypes 3, 6 and 7 has been designated CK-1G11-18.

As described above, human monoclonal antibodies to O-antigen of Pseudomonas aeruginosa have a very high activity as protective antibodies, as compared to human monoclonal antibodies to other antigens of Pseudomonas aeruginosa but are effective only for Pseudomonas aeruginosa infection of the corresponding serotype. According to the serological classification of the Serotyping Committee for the Japan Pseudomonas aeruginosa Society, O-antigen is classified into 13 groups and the serotype of Pseudomonas aeruginosa clinically isolated also covers all of these serotype bacteria, although there is a difference in frequency. Therefore, in order to generally use human monoclonal antibodies to O-antigen as a prophylactic and therapeutic agent against Pseudomonas aeruginosa infectious diseases, it is required to prepare a preparation by combining several to ten-odd kinds of antibodies. However, as the number of monoclonal antibodies to be used in combination increases, complexity in quality control of the preparation, etc. increases. In order to reduce such a complexity, it is desired to obtain human monoclonal antibodies to O-antigens showing cross reactivity with Pseudomonas aeruginosa of a plurality of different serotypes and having a potent protective activity against infectious diseases caused by Pseudomonas aeruginosa of a plurality of different serotypes. However, there is no proposal in the literature and no successful report on this, whilst it is also an important point to prepare a cell line capable of producing the aforesaid human monoclonal antibodies in a large quantity stably, which can be safely injected to human. Thus the object of the present invention is directed to these problems.

According to the invention this object is solved by the means of the claims.

Thus it is aimed at obtaining a cell line capable of secreting a single human monoclonal antibody having a high protective activity and capable of recognizing Pseudomonas aeruginosa having a plurality of serotypes in common; and it is obtained a self-reproducing cell line capable of continuously producing a human monoclonal antibody capable of reacting with a plurality of different O-antigens of Pseudomonas aeruginosa in common, namely, a human monoclonal antibody capable of plurally reacting with O-antigens of Pseudomonas aeruginosa and to achieve prophylaxis and therapy of Pseudomonas aeruginosa infectious diseases in human and animal using the human monoclonal antibody produced by this cell line singly or in combination as a prophylactic and therapeutic agent against Pseudomonas aeruginosa infectious diseases. Human antibody producing cells (B cells) are infected with Epstein-Barr virus (hereafter simply referred to as EB virus) to effect transformation (hereafter referred to as EB virus transformation); alternatively, human antibody producing cells are fused to human or animal derived cells capable of indefinite growth, for example, tumor cells, to obtain colonies capable of indefinite growth. From the colonies, a cell line capable of producing a single human monoclonal antibody having a reactivity with a plurality of Pseudomonas aeruginosa of different serotypes is selected by screening using the enzyme immunoassay and cloning. Next, this cell is cultured and a single human monoclonal antibody having a reactivity with Pseudomonas aeruginosa having different serotypes is purified from the culture solution in a conventional manner, for example, column chromatography, electrophoresis, precipitation, extraction, etc. The purified human monoclonal antibody can be provided as liquid preparations or lyophilized preparations for prophylaxis and therapy of Pseudomonas aeruginosa infectious diseases, singly or after adding optional additive thereto.

EP 0 327 648 B1

Brief Description of the Drawings

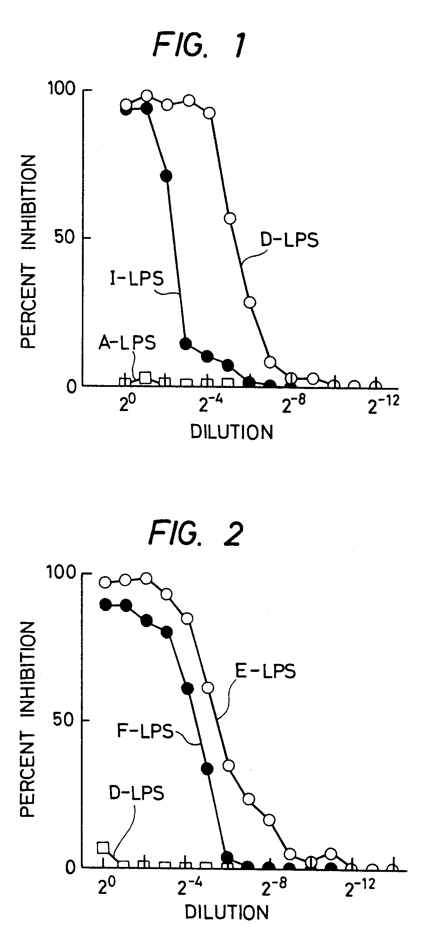

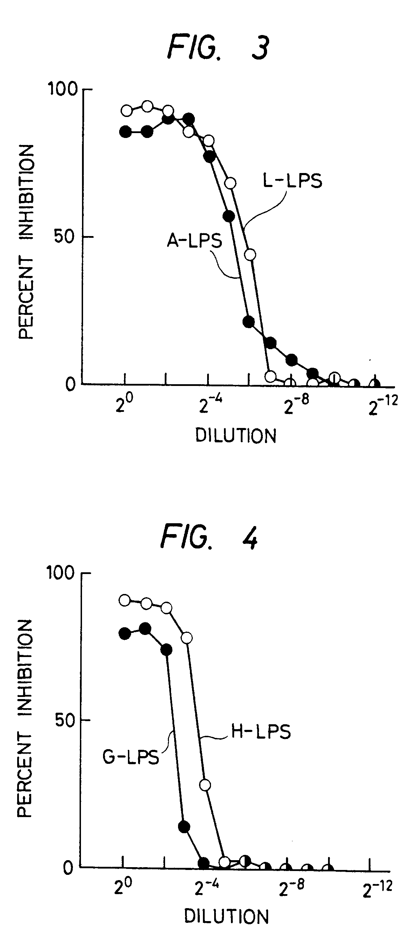

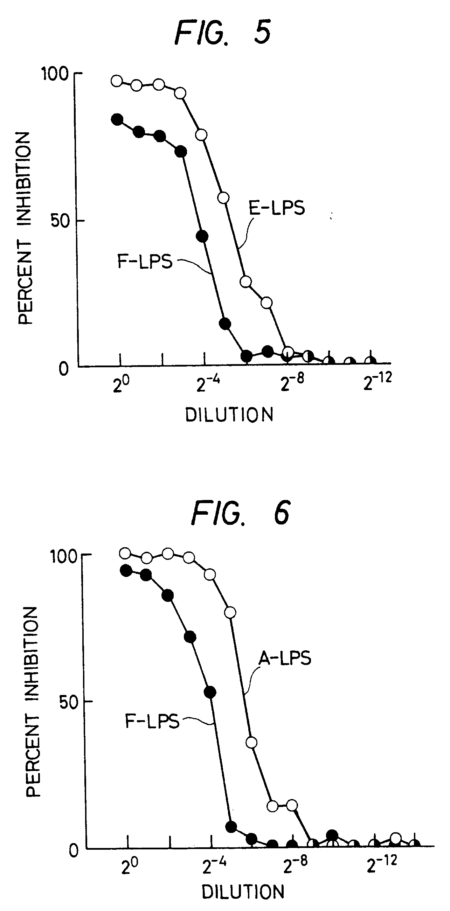

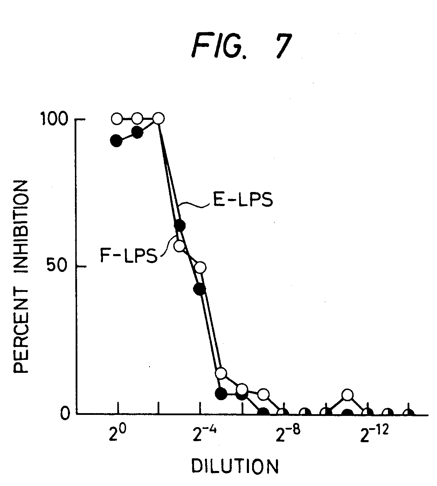

Figure 1 through 7 drawings show results obtained by examining cross reactivity of human monoclonal antibodies with O-antigens of Pseudomonas aeruginosa by inhibition tests of LPSs of a variety of serotype Pseudomonas aeruginosa on binding of human monoclonal antibody to LPS. Figure 1 shows LPSs of group D and group I Pseudomonas aeruginosa inhibit the binding of HPs1. Figure 2 shows LPSs of group E and group F Pseudomonas aeruginosa inhibit the binding of HPs2. Figure 3 shows LPSs of group A and group L Pseudomonas aeruginosa inhibit the binding of HPs4. Figure 4 shows LPSs of group G and group H Pseudomonas aeruginosa inhibit the binding of HPs5. Figure 5 shows LPSs of group E and group F Pseudomonas aeruginosa inhibit the binding of HPs6. Figure 6 shows LPSs of group A and group F Pseudomonas aeruginosa inhibit the binding of HPs7. Figure 7 shows LPSs of group E and group F Pseudomonas aeruginosa inhibit the binding of HPs8.

(Detailed Description)

1. Pseudomonas aeruginosa used

For purpose of convenience, classification of Pseudomonas aeruginosa used follows the serological classification of the Serotyping Committee for the Japan Pseudomonas aeruginosa Society in the present invention. Strains belonging to group A to group M which are shown in Table 1 are used.

Most strains belonging to group A to group M can be acquired from American Type Culture Collection (ATCC) and University of Tokyo, Institute of Medical Science.

2. Preparation of human monoclonal antibodies

The human monoclonal antibodies capable of plurally reacting with O-antigens of Pseudomonas aeruginosa, namely, human monoclonal antibodies to O-specific polysaccharide chain of LPS of Pseudomonas aeruginosa, according to the present invention can be prepared by the EB virus transformation method, the cell fusion method, etc.

The production of the human monoclonal antibodies according to the present invention by the EB virus transformation method can be performed by (1) preparing human antibody producing cells (human B cells), (2) infecting human antibody producing cells with EB virus to effect transformation, (3) detecting secretion of antibodies capable of reacting with plural Pseudomonas aeruginosa of different serotypes, (4) selecting a single cell line from the transformed cell colony (this operation is sometimes simply referred to as cloning hereinafter), (5) culturing the cell line and (6) purifying human monoclonal antibody from the culture.

Next, each step will be described in detail.

(1) Preparation of antibody producing cells

Human antibody producing cells (B cells) used in the method of the present invention can be obtained from peripheral blood, lymph node, tonsil or spleen collected from healthy donor capable of producing antibodies to Pseudomonas aeruginosa, or patient with Pseudomonas aeruginosa infectious disease past history, or from cord blood upon delivery, etc., by a known method. Isolation and concentration of human antibody producing cells (B cells) obtained from, for example, blood, tissues described above, etc. can be efficiently carried out by the density gradient centrifugation method using cell separation media such as Ficoll-Conray, etc., the E rosette formation method, the panning method, etc. in combination.

(2) Transformation

Transformation of human antibody producing cells (B cells) with EB virus can be carried out in a manner similar to known methods [for example, Nature, 269, 420-422 (1977)].

B95-8 cells (marmoset leucocyte derived cells capable of producing infectious EB virus) are cultured in 20% fetal calf serum (hereafter merely reffered to as FCS)-containing RPMI 1640 medium (hereafter sometimes merely referred to as culture solution). The culture supernatant on the 7th day close to the stationary phase is centrifuged to obtain a viral solution. [Proc. Jap. Soc. Immunol., 4, 399-401 (1974)]. Next, human antibody producing cells (B cells) obtained in (1) are centrifuged and the supernatant is removed by suction and the viral solution is added to the thus obtained pellets to disperse them, followed by incubation at 37°C for an hour in the presence of 5% carbon

6

dioxide. After incubation, centrifugation is performed. After removing the supernatant by suction, the culture solution is added to the pellets in a cell density of $1 \times 10^5$ to $5 \times 10^5$/ml to disperse the cells. The cell dispersion is separately charged in each well of a 24 well culture plate or a 96 well culture plate followed by incubation at 37°C for 2 to 4 weeks in the presence of 5% carbon dioxide. During the course of incubation, it is desired to replenish a half of the culture solution with a fresh culture solution.

(3) Detection of antibodies

Detection of the antibodies having reactivity with plural Pseudomonas aeruginosa of different serotypes can be performed by ordinary radio immunoassay, enzyme immunoassay, etc. [book entitled "Monoclonal Antibody", page 144, published by Kodansha Publishing Co., Ltd. (1983), and others]. In the present invention, the enzyme immunoassay is used. That is, there is used as a simple method the dot-immunobinding assay [hereafter simply referred to as DIBA, Anal. Biochem., 119, 142-147 (1982)] which comprises previously fixing 0.3% formalinized bacterial cells or LPSs of plural Pseudomonas aeruginosa of different serotypes to membrane filter, reacting the cell culture supernatant in a container for a definite time period, then reacting with enzyme conjugated rabbit anti-human antibody and determining the presence or absence of production and its amount of antibody produced by a degree in color formation of substrate through enzymatic reaction. If necessary, immunoglobulin type can be determined using an enzyme conjugated anti-human antibody specific to human immunoglobulin type.

(4) Cloning

A well in which the objective antibody is present is selected by testing the culture supernatant of each well where cell growth colonies have been noted, by the enzyme linked immunosorbent assay (hereafter simply referred to as ELISA method) described above, etc. Then, the cells in this well are subjected to cloning by the soft agar method [book entitled "Advanced Tissue Cultures", page 289, published by Soft Science Publishing Co. (1985), and others] or by the limiting dilution method [book entitled "Monoclonal Antibody", page 73, published by Kodansha Publishing Co., Ltd. (1983), and others]. Further after growth of the cells by cloning is noted, assay is again performed by ELISA described above. By cloning one to several times, a monoclonal cell line capable of secreting the objective antibody alone can be obtained.

(5) Culture of established cell line

The cell line established by the method of (4) can be cultured using ordinary medium. For example, the culture solution described above, ordinary low serum medium or serum free medium can be appropriately used.

(6) Purification of human monoclonal antibodies

Purification of the human monoclonal antibodies in accordance with the present invention from the culture solution can be performed by known methods in combination, for example, non-specific purification methods such as the ammonium sulfate precipitation method, the gel filtration method, the ion-exchange resin chromatography method, etc., the affinity chromatography method using a carrier having fixed thereto an antigen or a substance (for example, protein A, anti-human immunoglobulin antibody, etc.) having affinity to human monoclonal antibodies, or the like.

Production of the human monoclonal antibodies of the present invention according to the cell fusion method can be carried out in a manner similar to known methods [book entitled "MONOCLONAL ANTIBODIES", page 363, published by Plenum Press and others]. That is, the human monoclonal antibodies can be produced by (1) preparing human antibody producing cells (human B cells, etc.), (2) cell fusing the human antibody producing cells to cells capable of indefinite growth, (3) detecting secretion of antibodies capable of reacting with plural Pseudomonas aeruginosa of different serotypes, (4) selecting a single cell line from the hybridoma colonies, (5) culturing the cell line and then (6) purifying the human monoclonal antibodies from the culture.

(1) Preparation of antibody producing cells

As the antibody producing cells used for cell fusion, antibody producing cells similar to those as in the EB virus transformation method described above can be used. In addition, EB virus transformed cell

7

EP 0 327 648 B1

colonies obtained by the EB virus transformation method described above and capable of secreting the objective antibody prior to cloning, or a single EB virus transformed cell line obtained by cloning can also be used. Further, human antibody producing cells (B cells) are cultured in culture solution supplemented with pokeweed mitogen (PWM) for several days to proliferate the antibody producing cells, which can also be provided for cell fusion.

(2) Cell fusion

In production of human B cell hybridomas, it is preferred to use human derived myeloma cells, myeloma like cells, lymphoblast cells or lymphoblast like cells capable of indefinite growth and sensitive to culture solution containing hypoxanthine, aminopterine and thymidine (HAT medium) or culture solution containing hypoxanthine and azaserine (HA medium) as partner cells. Mouse myeloma cells, etc. sensitive to HAT medium can also be used as partner cells.

For example, mouse myeloma cells, P3-NSI/I-Ag4.1 (abbreviation, NS-I) as partner cells are mixed with cells of a well in which production of the objective antibody has been noted after transformation with EB virus or with antibody producing cells isolated from peripheral blood, etc. in a ratio of approximately 1 : 1 to 1 : 10. A medium (RPMI 1640 medium containing 50% polyethylene glycol and 10% dimethylsulfoxide, or the like) for cell fusion is added to the mixture followed by fusing cells by known methods. Then, the cells are dispersed in HAT-ouabain culture solution (culture solution further containing ouabain in the aforesaid HAT medium, HAT-O medium) suited for growth of the fused hybridoma alone in a cell density of $1 \times 10^5$ to $5 \times 10^6$/ml. The cell dispersion is separately charged in a 24 well or 96 well culture plate followed by incubation at 37°C for about 2 to about 4 weeks in the presence of 5% carbon dioxide. During course of the incubation, it is desired to replenish a half of the HAT-O medium with a fresh HAT-O medium every 3 to 5 days. In this case, when mouse peritoneal exudate cells, etc. are co-present as feeder cells, growth of the hybridoma can be accelerated.

Further the hybridoma can be produced by mixing human lymphoblast like cells, MHP-315 as partner with EB virus transformed cell line or antibody producing cells isolated from peripheral blood, etc. in a ratio of approximately 1 : 1 to 1 : 10, performing cell fusion as described above, then dispersing the cells in HA-ouabain culture solution (culture solution further containing ouabain in the aforesaid HA medium, HA-O medium) and culturing.

As the partner cells, there can be used mouse derived X63, P3 U1, X63.653, SP2/0, etc. and human derived SKO-007, GM1500, etc.

(3) Detection of antibodies

With respect to the well in which clear growth of the hybridoma has thus been noted, antibodies showing reactivity with plural Pseudomonas aeruginosa of different serotypes are detected by the enzyme immunoassay in a manner similar to the EB virus transformation method described above.

(4) Cloning

As stated in the EB virus transformation method described above, cloning is repeated to obtain a single cell line.

(5) Culture of established cell

In a manner similar to the EB virus transformation method described above, the single cell line can be cultured in a suitable culture solution.

(6) Purification of antibodies

In a manner similar to the EB virus transformation method described above, the human monoclonal antibodies of the present invention can be purified from the culture solution.

3. Production of human monoclonal antibody preparations

The human monoclonal antibodies capable of plurally reacting with O-antigens of Pseudomonas aeruginosa can be used as liquid preparations or lyophilized preparations singly or as admixture with

8

conventionally used additives or excipients. Upon actual use in prophylaxis and therapy of Pseudomonas aeruginosa infectious diseases, the human monoclonal antibodies of the present invention can be used singly or as a mixture of two or more monoclonal antibodies; alternatively, the human monoclonal antibodies may also be used as admixture with other human monoclonal antibodies against Pseudomonas aeruginosa and/or human globulin preparations. Particularly in the case of producing mixed preparations, the use of the present antibodies results in broadening a protection spectrum of the antibodies and therefore, the number of kinds of human monoclonal antibodies to be mixed can be reduced.

Dose and route for injection of the human monoclonal antibodies according to the present invention can be appropriately chosen but it is preferred that the dose be 0.01 to 10 mg/kg body weight and the route for injection can be intradermal, subcutaneous, intramuscular, intravenous, etc.

4. Examples

The present invention will be described in more detail with reference to the examples below.

Example 1 Production of human monoclonal antibodies by EB virus transformation technique I

(1) Preparation of antigen

Following the serological classification of the Serotyping Committee for the Japan Pseudomonas aeruginosa Society (group A to group M), bacterial cells and LPSs used for antibody assay were prepared from strains shown in Table 1 below.

Table 1

| Serological Classification of the Serotyping Committee for the Japan Pseudomonas aeruginosa Society | Strain* |
|---|---|
| A | Pseudomonas aeruginosa IID 1001(ATCC 27577) |
| B | IID 1002(ATTC 27578), IID 1007(ATCC 27583) IID 1013(ATCC 27589), IID 5004 |
| C | IID 1003(ATCC 27579) |
| D | IID 1004(ATCC 27580) |
| E | IID 1005(ATCC 27581), PA103 |
| F | IID 1006(ATCC 27582), F7 |
| G | IID 1008(ATCC 27584), P28 |
| H | IID 1009(ATCC 27585) |
| I | IID 1010(ATCC 27586) |
| J | IID 1011(ATCC 27587) |
| K | IID 1012(ATCC 27588) |
| L | IID 1014(ATCC 27590) |
| M | IID 5018 |

\* Among strains of Pseudomonas aeruginosa of

group A to group M exemplified in Table 1, ATCC

strains within parenthesis and IID strains have

been stored in American Type Culture Collection and University of Tokyo, Institute of Medical Science, respectively and are freely assigned to a third person.

Each Pseudomonas aeruginosa strain was cultured in nutrient agar (Nissui Pharmaceutical Co., Ltd.) at 37°C overnight. Proliferated colonies were collected and suspended in physiological salt solution to prepare a cell suspension. 0.5 ml of the cell suspension was inoculated in Sakaguti flask charged with 150 ml of Homma et al's synthetic medium [Tanamoto et al., J. Biochem., 83, 711-718 (1978)]per flask followed by shaking culture at 37°C for 16 hours. After incubation, formalin was added to the flask in a final concentration of 0.3% and the system was allowed to stand at room temperature for an hour. Lastly, the cells were collected by centrifugation (12,000 x g, 30 minutes). After washing with physiological salt solution and then distilled water in sequence, the cells were lyophilized to give formalinized dry cells. Each serotype Pseudomonas aeruginosa LPS was obtained using 20 g of formalinized wet cells prior to lyophilization as starting materials and purified by the hot phenol extraction method [book entitled "Meneki Jikken Sosaho" (Method for Immunological Experiments), page 2037, edited by Japanese Society of Immunology (1978)]. Yields (dry weight) of LPSs from formalinized cells of the respective serotype Pseudomonas aeruginosa were 25 to 75 mg.

(2) Preparation of EB viral solution

B95-8 cells producing and releasing EB virus were suspended in RPMI 1640 medium containing 20% FCS (hereafter sometimes referred to as culture solution) in a density of $3 \times 10^5$/ml followed by static culture at 37°C in the presence of 5% carbon dioxide. The culture supernatant on the 7th day close to the stationary phase was collected by centrifugation (800 x g, 10 minutes). After filtering through membrane filter (Millipore) having a pore size of 0.45 microns, the filtrate was used as EB viral solution for transformation experiment.

(3) Preparation of human antibody producing cells (lymphocytes)

An equal amount of RPMI 1640 medium was added to 50 ml of heparinized peripheral blood collected from healthy donor to dilute to 2-fold. Then, the dilution was laid on a Ficoll-Paque (Pharmacia) of a half amount of the dilution so as not to disturb the interface followed by centrifugation (400 x g, 30 minutes) at room temperature. After the centrifugation, the interface phase was taken out using a pasteur pipette and an equal amount of 20% FCS-containing RPMI 1640 medium was added thereto. The mixture was centrifuged (250 x g, 10 minutes) at room temperature. The precipitated cells were suspended in 20% FCS-containing RPMI 1640 medium. Centrifugal operation was further repeated once to obtain pellet (cell count $5 \times 10^7$) of human antibody producing cells (lymphocytes).

(4) Transformation with EB virus

To $5 \times 10^7$ of human antibody producing cells was added 50 ml of the viral solution prepared in (2) followed by incubation at 37°C for an hour. After incubation, the cells were collected by centrifugation (250 x g, 10 minutes). The cells were dispersed in 20% FCS-containing RPMI 1640 medium. After adjusting the density to $5 \times 10^5$/ml, 0.1 ml each of the suspension was added to a 96 well flat bottom culture plate followed by static culture at 37°C in the presence of 5% carbon dioxide. Four days after, 0.1 ml of 20% FCS-containing RPMI 1640 medium was added to the culture and thereafter, a half of the culture solution was replaced with a fresh culture solution every 4 or 5 days. By the antibody detection method shown in (5), cells of a well in which the reactivity with Pseudomonas aeruginosa was noted were cultured in a 24 well culture plate on an enlarged scale.

(5) Detection of antibodies

With respect to the well in which cell growth was noted, the presence or absence of human monoclonal antibodies to Pseudomonas aeruginosa in the culture supernatant was determined by the DIBA method classified into the enzyme-linked immunosorbent assay. In the case of the culture supernatant in the 96 well flat bottom culture plate, 0.1 ml of the culture supernatant in each well was reacted with grid printed nitrocellulose membrane filter (3.1 mm square) having fixed a mixture of 0.4 $\mu$g/dot each of formalinized dry cells from Pseudomonas aeruginosa of 13 serotypes in a 96 well U-shaped bottom multiplate. In the case of the culture supernatant in the 24 well culture plate, 0.2 ml of the culture supernatant in each well was reacted in a 48 well culture plate with grid-printed nitrocellulose membrane filters (6.2 mm x 9.3 mm square) having fixed formalinized dry cells from Pseudomonas aeruginosa of 6 kinds of group A to group F and 7 kinds of group G to group M on different places. After reacting at room temperature for 2 hours and then reacting with peroxidase-conjugated rabbit anti-human immunoglobulin (DAKO) for 2 hours, color was formed using 4-chloro-1-naphthol as a substrate. Antibody production was judged to be positive with those in which color formation was noted on the antigen-fixed nitrocellulose membrane filter by observation with the naked eye.

(6) Cloning

The cells of a well in which the reactivity with plural Pseudomonas aeruginosa of different serotypes was noted by the antibody detection method were transferred to a 6 cm dish. The cells proliferated on the 6 cm dish were cloned by the soft agar method. Firstly, after accurately counting the cells with a hemacytometer, the cells were made a cell suspension in a density of 1 x 10$^6$/ml and 0.1 ml of the cell suspension was added to 30 ml of a culture solution containing 0.3% agarose (Sea Plaque agarose, FMC) followed by mixing them. Next, 3 ml of the culture solution containing the cells and 0.3% agarose was separately charged in a 6 cm dish, which had been solidified by previously separately charging 4 ml of the culture solution containing 0.5% agarose, to solidify (10 dishes per each well). The 6 cm dish in which the cells had been separately charged was subjected to static culture at 37°C in the presence of 5% carbon dioxide. Three to five weeks after, the cells grew in soft agar and colonies were recognized with the naked eye; at this stage, each colony was transferred with a pasteur pipette to and cultured in each well of a 96 well flat bottom culture plate in which 0.1 ml/well of the culture solution had been separately charged. Two days after, 0.1 ml of the culture solution was added and further 2 days after, the presence or absence of human monoclonal antibodies to Pseudomonas aeruginosa in the culture supernatant was determined by the DIBA method, with respect to the well in which cell growth was noted. Cells in a well in which antibody production was judged to be positive were cultured in a 24 well culture plate on an enlarged scale. Three days after, the presence or absence of human monoclonal antibodies to Pseudomonas aeruginosa in the culture supernatant was determined by the DIBA method, with respect to the wells in 24 well culture plate. Operations similar to above were again repeated as described above, with cells in a well which was judged to produce an antibody capable of reacting with Pseudomonas aeruginosa of a plurality of serotypes, to effect cloning. Thus, EB virus transformed cell line MP 5035 capable of producing human monoclonal antibody HPs1 (IgM) having cross reactivity with group I and group D of serotype in the serological classification of the Serotyping Committee for the Japan Pseudomonas aeruginosa Society, and EB virus transformed cell line MP 5038 capable of producing human monoclonal antibody HPs2 (IgM) having cross reactivity with group E and group F of serotype in the serological classification of the Serotyping Committee for the Japan Pseudomonas aeruginosa Society, were obtained. MP 5035 and MP 5038 have been deposited in the Fermentation Research Institute of the Agency of Industrial Science and Technology of Japan under FERM BP-1598 and FERM BP-1596.

(7) Culture of cell line and purification of antibodies

Two EB virus transformed cell lines capable of producing human monoclonal antibodies HPs1 and HPs2 respectively were cultured in RITC 55-9 medium [Exp. Cell Res., 138, 127-134 (1982)] containing 0.5% bovine serum albumin (hereafter merely referred to as BSA) using a flask (bottom area of 175 cm$^2$). The culture was centrifuged (400 x g, 30 minutes) to give the supernatant and, the antibodies were purified by the 50% ammonium sulfate precipitation method and the fractionation method using Sephacryl S-300 (Pharmacia) column. From 250 ml of the culture solution obtained by culturing the EB virus transformed cell line capable of producing HPs1, 2.5 mg of IgM fraction was obtained and 76 mg of IgM fraction was obtained from 2 liters of the culture solution obtained by culturing the EB virus transformed cell line capable of producing HPs2.

Example 2 Production of human monoclonal antibodies by EB virus transformation technique II

Transformation with EB virus was carried out in a manner similar to Example 1 except for using peripheral blood collected from patients with Pseudomonas aeruginosa infectious disease. Pellet (cell count; $1.4 \times 10^7$) of human antibody producing cells were obtained from 25 ml of heparinized peripheral blood. Soft agar cloning was conducted twice to give EB virus transformed cell line MP 4091 capable of producing human monoclonal antibody HPs4 (IgM) having cross reactivity with group A and group L of serotype in the serological classification of the Serotyping Committee for the Japan Pseudomonas aeruginosa Society, and EB virus transformed cell line MP 5050 capable of producing human monoclonal antibody HPs5 (IgM) having cross reactivity with group G and group H of serotype in the serological classification of the Serotyping Committee for the Japan Pseudomonas aeruginosa Society. MP 5050 has been deposited in the Fermentation Research Institute of the Agency of Industrial Science and Technology of Japan under FERM BP-1600. The cells were cultured in a manner similar to Example 1 (7). From 250 ml of the culture solution obtained by culturing the EB virus transformed cell line MP 4091 capable of producing HPs4, 2.5 mg of IgM fraction was obtained. From 450 ml of the culture solution obtained by culturing the EB virus transformed cell line MP 5050 capable of producing HPs5, 8.6 mg of IgM fraction was obtained.

Example 3 Production of human monoclonal antibody by EB virus transformation technique III

Transformation with EB virus was carried out in a manner similar to Example 1 except for using peripheral blood collected from another healthy donor. Pellet (cell count; $2.8 \times 10^7$) of human antibody producing cells were obtained from 25 ml of heparinized peripheral blood. Soft agar cloning was conducted twice to give EB virus transformed cell line MP 5046 capable of producing human monoclonal antibody HPs7 (IgM) having cross reactivity with group A and group F of serotype in the serological classification of the Serotyping Committee for the Japan Pseudomonas aeruginosa Society. MP 5046 has been deposited in the Fermentation Research Institute of the Agency of Industrial Science and Technology of Japan under FERM BP-1599. The cells were cultured in a manner similar to Example 1 (7). From 300 ml of the culture solution obtained by culturing the EB virus transformed cell line MP 5046 capable of producing HPs7, 4.1 mg of IgM fraction was obtained.

Example 4 Production of human monoclonal antibody by hybridoma technique I

(1) Preparation of human antibody producing cells (B cells)

Pellet (cell count; $4.6 \times 10^7$) of human antibody producing cells were obtained from 50 ml of heparinized peripheral blood collected from healthy donor, in a manner similar to Example 1 (3). To the pellet was added 46 ml of the viral solution prepared in Example 1 (2) followed by incubation at 37°C for an hour. After incubation, a culture solution was added to the cells collected by centrifugation (250 x g, 10 minutes) to suspend the cells in a density of $1 \times 10^5$/ml. The suspension was separately charged in two flasks (bottom area of 175 cm²) followed by static culture at 37°C in the presence of 5% carbon dioxide. Five days after 50 ml each of the culture solution was added and further 5 days after, the cells were collected by centrifugation.

(2) Cell fusion

EB virus transformed cells and mouse myeloma cell line NS-1 were washed with RPMI 1640 medium, respectively. In a plastic-made centrifugal tube of a 50 ml volume $1.3 \times 10^8$ of EB virus transformed cells and $1.3 \times 10^8$ of mouse myeloma cells were mixed with each other. The mixed cells were centrifuged (175 x g, 10 minutes). The supernatant was discarded and the cells were loosened by gentle vibration. To the centrifugal tube containing the cells, 0.5 ml of RPMI 1640 medium supplemented with 50% polyethylene glycol (M.W. 1500, Wako Junyaku) and 10% dimethylsulfoxide was gently added. While slowly rotating the centrifugal tube, the cells were fused. Two minutes after, 10 ml of RPMI 1640 medium was added thereto. After gently agitating, centrifugation (175 x g, 10 minutes) was performed. After the centrifugation, the supernatant was discarded and RPMI 1640 medium containing $2 \times 10^{-4}$ M hypoxanthine, 0.176 $\mu$g/ml of aminoputerine, 13 $\mu$g/ml of thymidine, 5 $\mu$M ouabain and 20% FCS (hereafter merely referred to as HAT-O culture solution) was added to make a cell suspension in a density of $1 \times 10^6$/ml. 0.1 ml each per well of the

EP 0 327 648 B1

suspension was charged in a 96 well flat bottom culture plate followed by static culture at 37°C in the presence of 5% carbon dioxide. Four days after 0.1 ml of the HAT-O culture solution was added and thereafter, a half of the culture solution was replenished with a fresh HAT-O culture solution every 4 to 5 days.

(3) Detection of antibody

With respect to the well in which cell growth was noted, the presence or absence of human monoclonal antibody to Pseudomonas aeruginosa in the culture supernatant was determined by the DIBA method in a manner similar to Example 1 (5). The culture supernatant, 0.1 ml, in a 96 well flat bottom culture plate was diluted with RPMI 1640 medium to 2-fold and reacted in a 48 well culture plate with two grid-printed nitrocellulose membrane filters (6.2 mm x 9.3 mm square) having fixed formalinized dry cells from Pseudomonas aeruginosa of 6 kinds of group A to group F and 7 kinds of group G to group M on different places.

(4) Cloning

3% Glycogen (Tokyo Kasei Kogyo)-containing phosphate buffered saline (hereafter simply referred to as PBS) was previously injected intraperitoneally to mice (Balb/c) and peritoneal exudate cells were collected 4 days after to make a cell suspension in a density of $1 \times 10^5$/ml and a 96 well flat bottom culture plate separately charged with 0.1 ml each/well of the cell suspension was prepared. Next, cells in wells in which reactivity with Pseudomonas aeruginosa of a plurality of serotypes was noted by the antibody detection method were collected, respectively. The cells were accurately counted using a hemacytometer and dispersed in HAT-O culture solution to make cell suspensions of 20/ml and 200/ml. After the supernatant of each well in the 96 well flat bottom culture plate separately charged with the mouse peritoneal exudate cells described above was removed, 0.1 ml each was added followed by static culture at 37°C in the presence of 5% carbon dioxide. Four days after 0.1 ml of the HAT-O culture solution was added and thereafter, a half of the culture solution was replenished with a fresh HAT-O culture solution every 4 to 5 days. With respect to wells in which cell growth was noted 7 to 14 days after, the presence or absence of human monoclonal antibody to Pseudomonas aeruginosa in the culture supernatant was determined by the DIBA method. Cells in a well which was judged to produce an antibody capable of reacting with Pseudomonas aeruginosa of a plurality of serotypes were again repeatedly subjected to operations similar to above thereby to achieve cloning. Thus, hybridoma cell line MP 4092 capable of producing human monoclonal antibody HPs6 (IgG) cross reactive with each of group E and group F in the serological classification of the Serotyping Committee for the Japan Pseudomonas aeruginosa Society, was obtained.

(5) Culture of cell line and purification of antibody

The hybridoma cells thoroughly grown in a 96 well flat bottom culture plate were cultured on a gradually enlarged scale, and in the stirring system of 1 liter, culture was performed using 500 ml of 20% FCS-containing RPMI 1640 medium. The culture was centrifuged (400 x g, 30 minutes) to give the supernatant and, the antibody was purified by the 50% ammonium sulfate precipitation method and the fractionation method using Protein A-Sepharose 4B (Pharmacia) column to give 5 mg of IgG.

Example 5 Production of human monoclonal antibody by hybridoma technique II

(1) Preparation of 2-aminoethyl isothiouronium hydrobromide (hereafter simply referred to as AET)-treated sheep red blood cells

In a plastic-made centrifugal tube of a 50 ml volume was taken 5 ml of a suspension of sheep red blood cells (Nihon Biotest Laboratory, hereafter simply reffered to as SRBC) and, 20 ml of gelatin veronal buffer (hereafter simply referred to as GVB) was added to the suspension. After thoroughly agitating, the mixture was centrifuged (1,600 x g, 10 minutes). The supernatant was discarded by suction and precipitated SRBC was suspended in 20 ml of GVB. Centrifugal operation was repeated further 3 times. To about 1 ml of SRBC pellet obtained was added 4 ml of 0.143 M AET aqueous solution to suspend the pellets. While agitating sometimes, the suspension was reacted at 37°C for 15 minutes. Immediately after completion of the reaction, 20 ml of ice-cooled GVB was added to the reaction mixture. After thorough agitation,

14

centrifugation was carried out (1,600 x g, 10 minutes). The supernatant was discarded by suction and the precipitates were suspended in 20 ml of GVB. Centrifugal operation was repeated further twice. The AET-treated SRBC was suspended in GVB in a density of $2 \times 10^8$/ml and stored under ice cooling until it was provided for use.

(2) Preparation of human antibody producing cells (B cells)

On an equal amount of mono-poly resolving medium (Flow) was laid 25 ml of heparinized peripheral blood collected from healty donor having a high titer to Pseudomonas aeruginosa who was in hospital by car accident and passed for about 3 months after cure of wound so as not to disturb the interface followed by centrifugation (350 x g, 30 minutes) at room temperature. After the centrifugation, liquid containing cells at the interface was taken out using a pasteur pipette and an equal amount of 20% FCS-containing RPMI 1640 medium was added thereto. The mixture was centrifuged (350 x g, 10 minutes) at room temperature. The precipitated cells were again suspended in 20% FCS-containing RPMI 1640 medium. Centrifugal operation was further repeated once to obtain pellet (cell count $5 \times 10^7$) of human lymphocyte fraction.

Next, the obtained human lymphocyte fraction was suspended in 4 ml of 20% FCS-containing RPMI 1640 medium to make a suspension in a density of $5 \times 10^6$/ml. In 8 plastic-made centrifugal tubes of a 50 ml each volume was separately charged 0.5 ml each of the suspension and, 1 ml each of the AET-treated SRBC suspension prepared in (1) was added to each centrifugal tube. After thoroughly agitating, the mixture was centrifuged (20 x g, 2 minutes). After the centrifugation, the system was settled at 4°C for 2 hours to perform rosette formation. After completion of the reaction, 1.5 ml of 20% FCS-containing RPMI 1640 medium was added and the pellet was gently destroyed using a pasteur pipette. The cell suspensions which formed rosette were collected and laid on 20 ml of mono-poly resolving medium charged in a plastic-made centrifugal tube of a 50 ml volume so as not to disturb the interface followed by centrifugation (350 x g, 30 minutes). After the centrifugation, liquid containing cells at the interface was taken out using a pasteur pipette and an equal amount of 20% FCS-containing RPMI 1640 medium was added thereto. The mixture was centrifuged (250 x g, 10 minutes) at room temperature. The precipitated cells were again suspended in 20% FCS-containing RPMI 1640 medium. Centrifugal operation was repeated further twice to obtain pellet (cell count $1.0 \times 10^7$) of human antibody producing cells (B cells).

The pellet of human antibody producing cells (B cells) was suspended in 20 ml of 20% FCS-containing RPMI 1640 medium. The suspension was adjusted to a density of $5 \times 10^5$/ml. Pokeweed mitogen (PWM) (Gibco) of a 1/200 volume was added to the suspension. The mixture was charged in a flask (bottom area of 75 cm$^2$) followed by static culture at 37°C for 6 days in the presence of 5% carbon dioxide.

3) Cell fusion

PBS containing 2.5% glycogen was previously injected intraperitoneally to mice (Balb/c) in 0.5 ml/mouse and peritoneal exudate cells were collected 4 days after. The cells were suspended in 20% FCS-containing RPMI 1640 medium, together with mouse spleen cells separately prepared to make cell suspensions in densities of $5 \times 10^5$/ml and $1 \times 10^6$/ml, respectively. A 96 well flat bottom culture plate (hereafter simply referred to as feeder plate) separately charged with 0.1 ml each/well of each cell suspension was prepared.

PWM-treated human antibody producing cells (B cells) and human lymphoblast like cells MHP-315 were washed with RPMI 1640 medium, respectively. In a plastic-made centrifugal tube of a 50 ml volume 5 $\times 10^7$ of human antibody producing cells (B cells) and the same count of human lymphoblast like cells were mixed with each other. The mixed cells were centrifuged (175 x g, 10 minutes). The supernatant was discarded and the cells were suspended in FCS free RPMI 1640 medium. Centrifugal operation was repeated further twice. After the centrifugation, the supernatant was removed using a pasteur pipette as much as possible to make cell pellet. Gentle vibration was given to the pellet to loosen the cells a little. Then 0.3 ml of a fusing reagent (PBS containing 50% polyethylene glycol 4000 and 10% dimethylsulfoxide) was added thereto. The centrifugal tube was slowly rotated for 90 seconds. Ninety seconds after, 20 ml of 20% FCS-containing RPMI 1640 medium was gradually added thereto. Further 2 minutes after, 20 ml of the same medium was added to gently disperse the cells. The cells after cell fusion was pelletized by centrifugation (175 x g, 10 minutes). The pellet was dispersed in 20% FCS-containing RPMI 1640 medium to adjust a density of $1 \times 10^6$/ml. After the supernatant in each well of the feeder plate was removed, 0.1 ml each/well of the cell dispersion was added followed by static culture at 37°C in the presence of 5% carbon dioxide.

Twenty four hours after, 0.1 ml each/well of 20% FCS-containing RPMI 1640 medium containing 4 x $10^{-4}$ M hypoxanthine, 2 $\mu$g/ml of azaserine and 10 $\mu$M ouabain was added to the culture. Thereafter, a half of the culture solution was replenished with 20% FCS-containing RPMI 1640 medium containing 2 x $10^{-4}$ M hypoxanthine, 1 $\mu$g/ml of azaserine and 5 $\mu$M ouabain (hereafter simply referred to as HA-O culture solution) every 3 to 4 days.

(4) Detection of antibody

With respect to the well in which cell growth was noted about 4 to 5 weeks after, the presence or absence of human monoclonal antibody to Pseudomonas aeruginosa in the culture supernatant was determined by the DIBA method in a manner similar to Example 1 (5).

(5) Cloning

Cells in wells in which reactivity with Pseudomonas aeruginosa of a plurality of serotypes was noted by the antibody detection method were collected, respectively. The cells were accurately counted using a hemacytometer and dispersed in HA-O culture solution to make cell suspensions of 20/ml and 200/ml. After the supernatant of each well in the feeder plate described above was removed, 0.1 ml each was added followed by static culture at 37°C in the presence of 5% carbon dioxide. Four days after 0.1 ml of the HA-O culture solution was added and thereafter, a half of the culture solution was replenished with a fresh HA-O culture solution every 3 to 4 days. With respect to the well in which cell growth was noted about 2 to 4 weeks after, the presence or absence of human monoclonal antibody to Pseudomonas aeruginosa in the culture supernatant was determined by the DIBA method. Cells which were judged to produce an antibody reactive with Pseudomonas aeruginosa of a plurality of serotypes were again subjected to operations similar to above thereby to achieve cloning. Thus, hybridoma cell line MP 4095 capable of producing human monoclonal antibody HPs8 (IgM) cross reactive with each of group E and group F in the serological classification of the Serotyping Committee for the Japan Pseudomonas aeruginosa Society, was obtained. MP 4095 has been deposited in the Fermentation Research Institute of the Agency of Industrial Science and Technology of Japan under FERM P-9750.

(6) Culture of cell line and purification of antibody

The hybridoma cell line MP 4095 thoroughly grown in a 96 well flat bottom culture plate was cultured on a gradually enlarged scale, and was cultured on 4 flasks (bottom area of 175 cm²) using 200 ml of NYSF 404 serum free medium [Yabe, Tissue Culture, 11, 458 (1985)]. The culture was centrifuged (400 x g, 30 minutes) to give 190 ml of the supernatant. The supernatant was filtered through membrane filter having a pore size of 0.22 microns. The filtrate was adsorbed to Mono Q column (Pharmacia) at a flow rate of 2 ml/min, which column had been previously equilibrated with 0.025 M phosphate buffer (pH 6.8). After adsorption, the column was washed with a mixture of the same amounts of said buffer and 0.3 M phosphate buffer (pH 6.5). After washing, IgM fraction was eluted with 0.3 M phosphate buffer (pH 6.5). From 190 ml of the culture supernatant, 1 mg of IgM antibody was obtained.

Example 6 Production of human monoclonal antibody by hybridoma technique III

(1) Preparation of human antibody producing cells

As human antibody producing cells, EB virus transformed cell line MP 5035 capable of producing human monoclonal antibody HPs1 (IgM) obtained in Example 1 and having cross reactivity with group I and group D in the serological classification of the Serotyping Committee for the Japan Pseudomonas aeruginosa Society was used. MP 5035 was cultured in a flask (bottom area of 175 cm²) using 20% FCS-containing RPMI 1640 medium. Cells in the logarithmic phase were collected by centrifugation and provided to produce hybridoma.

(2) Cell fusion

The transformed cell line MP 5035 and human lympho-blast like cell line MHP-315 were washed with FCS free RPMI 1640 medium, respectively. In a plastic-made centrifugal tube of a 50 ml volume 1.3 x $10^8$ of the transformed cells and the same count of human lymphoblast like cells were mixed with each other.

The mixed cells were centrifuged (175 x g, 10 minutes). The supernatant was discarded and the precipitated cells were suspended in FCS free RPMI 1640 medium. Centrifugal operation was repeated further twice. After the centrifugation, the supernatant was removed using a pasteur pipette as much as possible to make cell pellet. Gentle vibration was given to the pellet to loosen the cells a little. Then 0.3 ml of a fusing reagent (PBS containing 50% polyethyleneglycol 4000 and 10% dimethylsulfoxide) was added thereto. The centrifugal tube was slowly rotated for 90 seconds at room temperature. Ninety seconds after, 20 ml of 20% FCS-containing RPMI 1640 medium was was gradually added thereto. Further 2 minutes after, 20 ml of the same medium was added to gently disperse the cells. The cells after cell fusion was pelletized by centrifugation (175 x g, 10 minutes). The pellet was dispersed in 20% FCS-containing RPMI 1640 medium to adjust a density of $1 \times 10^6$/ml. After the supernatant in each well of the feeder plate was removed, 0.1 ml each/well of the cell dispersion was added followed by static culture at 37°C in the presence of 5% carbon dioxide.

Twenty four hours after, 0.1 ml each/well of 20% FCS-containing RPMI 1640 medium containing 4 x $10^{-4}$M hypoxanthine, 2 $\mu$g/ml of azaserine and 10 $\mu$M ouabain was added to the culture. Thereafter, a half of the culture solution was replenished with 20% FCS-containing RPMI 1640 medium containing 2 x $10^{-4}$M hypoxanthine, 1 $\mu$g/ml of azaserine and 5 $\mu$M ouabain (HA-O culture solution) every 3 to 4 days.

(3) Detection of antibody

With respect to the well in which cell growth was noted about 4 to 5 weeks after, the presence or absence of human monoclonal antibody to Pseudomonas aeruginosa in the culture supernatant was determined by the DIBA method in a manner similar to Example 1 (5).

(4) Cloning

Cells in wells in which the presence of an antibody having reactivity with Pseudomonas aeruginosa of serotypes of group D and group I was noted by the antibody detection method were collected, respectively. The cells were accurately counted using a hemacytometer and dispersed in HA-O culture solution to make cell suspensions of 20/ml and 200/ml. After the supernatant of each well in the feeder plate (each one plate) was removed, 0.1 ml each was inoculated per each well followed by static culture at 37°C in the presence of 5% carbon dioxide. Four days after 0.1 ml of the HA-O culture solution was added and thereafter, a half of the culture solution was replenished with a fresh HA-O culture solution every 3 to 4 days. With respect to the well in which cell growth was noted about 2 to 4 weeks after, the presence or absence of human monoclonal antibody to Pseudomonas aeruginosa in the culture supernatant was determined by the DIBA method. Cells which were judged to produce an antibody reactive with Pseudomonas aeruginosa of serotypes of group D and group I were again subjected to operations similar to above thereby to achieve cloning. Thus, hybridoma cell line MP 5082 capable of producing human monoclonal antibody HPs9 (IgM) cross reactive with each of group D and group I in the serological classification of the Serotyping Committee for the Japan Pseudomonas aeruginosa Society, was obtained with twice cloning. The cells thoroughly grown in a 96 well flat bottom culture plate were cultured on a gradually enlarged scale. MP 5082 has been depodited in the Fermentation Research Institute of the Agency of Industrial Science and Technology of Japan under FERM P-9745.

(5) Culture of cell line and purification of antibody

The hybridoma cell line MP 5082 was dispersed in 200 ml of NYSF 404 serum free medium in a cell density of $3 \times 10^5$/ml. The suspension was separately charged in 4 flasks (bottom area of 175 cm$^2$) followed by static culture at 37°C in the presence of 5% carbon dioxide. On the fourth day after onset of the culture, the culture solution was centrifuged (400 x g, 20 minutes) to give 190 ml of the supernatant. From the culture supernatant, the antibody was purified by Mono Q column in a manner similar to Example 5 (6). From 190 ml of the culture supernatant, 1.7 mg of IgM antibody was obtained.

Example 7 Production of human monoclonal antibody by hybridoma technique IV

Cell fusion was performed between human antibody producing cells and human derived lymphoblast like cell line MHP-315 in a manner similar to Example 6, except for using, as human antibody producing cells, EB virus transformed cell line MP 5038 established in Example 1, capable of producing human monoclonal antibody HPs2 (IgM) and having cross reactivity with group E and group F in the serological classification of the Serotyping Committee for the Japan Pseudomonas aeruginosa Society. Firstly, MP 5038 was cultured in a flask (bottom area of 175 cm$^2$) using 20% FCS-containing RPMI 1640 medium. Cells in the logarithmic phase were collected by centrifugation and provided to produce hybridoma. Cell fusion was performed between 1.0 x 10$^8$ of the EB virus transformed cells and the same count of human lymphoblast like cells followed by cloning in a manner similar to Example 6. Thus, there was obtained hybridoma cell line MP 5064 capable of producing human monoclonal antibody HPs10 (IgM) cross reactive with each of group E and group F in the serological classification of the Serotyping Committee for the Japan Pseudomonas aeruginosa Society. MP 5064 has been deposited in the Fermentation Research Institute of the Agency of Industrial Science and Technology of Japan under FERM P-9751. The hybridoma cell line MP 5064 was dispersed in 350 ml of NYSF 404 serum free medium in 3 x 10$^5$/ml. The suspension was separately charged in 7 flasks (bottom area of 175 cm$^2$) followed by static culture at 37°C in the presence of 5% carbon dioxide. On the fourth day after onset of the culture, the culture solution was centrifuged (400 x g, 20 minutes) to give 330 ml of the supernatant. From the culture supernatant, the antibody was purified by Mono Q column in a manner similar to Example 5 (6). From 300 ml of the culture supernatant, 7.7 mg of IgM was obtained.

Example 8 Production of human monoclonal antibody by hybridoma technique V

Cell fusion was performed between human antibody producing cells and human derived lymphoblast like cell line MHP-315 in a manner similar to Example 6, except for using, as human antibody producing cells, EB virus transformed cell line MP 5050 established in Example 2, capable of producing human monoclonal antibody HPs5 (IgM) and having cross reactivity with group G and group H in the serological classification of the Serotyping Committee for the Japan Pseudomonas aeruginosa Society. Firstly, MP 5050 was cultured in a flask (bottom area of 175 cm$^2$) using 20% FCS-containing RPMI 1640 medium. Cells in the logarithmic phase were collected by centrifugation and provided to produce hybridoma. Cell fusion was performed between 1.3 x 10$^8$ of the EB virus transformed cells and the same count of human lymphoblast like cells followed by a manner similar to Example 6. Thus, there was obtained hybridoma cell line MP 5104 capable of producing human monoclonal antibody HPs11 (IgM) cross reactive with each of group G and group H in the serological classification of the Serotyping Committee for the Japan Pseudomonas aeruginosa Society. MP 5104 has been deposited in the Fermentation Research Institute of the Agency of Industrial Science and Technology of Japan under FERM P-9753. The hybridoma cell line MP 5104 was dispersed in 500 ml of NYSF 404 serum free medium in 3 x 10$^5$/ml. The suspension was separately charged in 10 flasks (bottom area of 175 cm$^2$) followed by static culture at 37°C in the presence of 5% carbon dioxide. On the fourth day after onset of the culture, the culture solution was centrifuged (400 x g, 20 minutes) to give 470 ml of the supernatant. From the culture supernatant, the antibody was purified by Mono Q column in a manner similar to Example 5 (6). From 450 ml of the culture supernatant, 9.8 mg of IgM antibody was obtained.

Example 9 Production of human monoclonal antibody by hybridoma technique VI

Cell fusion was performed between human antibody producing cells and human derived lymphoblast like cell line MHP-315 in a manner similar to Example 6, except for using, as human antibody producing cells, EB virus transformed cell line MP 5046 established in Example 3, capable of producing human monoclonal antibody HPs7 (IgM) and having cross reactivity with group A and group F in the serological classification of the Serotyping Committee for the Japan Pseudomonas aeruginosa Society. Firstly, MP 5046 was cultured in a flask (bottom area of 175 cm$^2$) using 20% FCS-containing RPMI 1640 medium. Cells in the logarithmic phase were collected by centrifugation and provided to produce hybridoma. Cell fusion was performed between 1.3 x 10$^8$ of the EB virus transformed cells and the same count of human lymphoblast like cells followed by a manner similar to Example 6. Thus, there was obtained hybridoma cell line MP 5075 capable of producing human monoclonal antibody HPs12 (IgM) cross reactive with each of group A and group F in the serological classification of the Serotyping Committee for the Japan Pseudomonas aeruginosa Society. MP 5075 has been deposited in the Fermentation Research Institute of the Agency of Industrial Science and Technology of Japan under FERM P-9752. The hybridoma cell line MP 5075 was dispersed in 550 ml of NYSF 404 serum free medium in 3 x 10$^5$/ml. The suspension was separately charged in 11 flasks (bottom area of 175 cm$^2$) followed by static culture at 37°C in the presence of 5% carbon dioxide. On the fourth day after onset of the culture, the culture solution was centrifuged (400 x g, 20 minutes) to give 530 ml of the supernatant. From the culture supernatant, the antibody was purified by Mono Q column in a manner similar to Example 5 (6). From 530 ml of the culture supernatant, 13.4 mg of IgM was obtained.

Example 10 Reactivity of human monoclonal antibodies with LPS, Ra and Lipid A

Reactivities of the human monoclonal antibodies obtained in Examples 1, 2, 3, 4, 5, 6, 7, 8 and 9 with LPS of each serotype of Pseudomonas aeruginosa, Ra strain derived LPS and lipid A having only a common structural region of LPS were examined by the DIBA method described in Example 1. As each serotype Pseudomonas aeruginosa LPS, one prepared in Example 1 was used. As Ra (Salmonella minnesota R60) strain derived LPS and lipid A, those commercially available (List Biological Laboratories Inc.) were used. The results are shown in Table 2. Each human monoclonal antibody did not react with Ra or lipid A which is a common structural region of LPS but specifically reacted with each serotype Pseudomonas aeruginosa LPS. Therefore, it was made clear that they were human monoclonal antibodies of recognizing O-specific polysaccharide chain of LPS.

Table 2

| Monoclonal Antibody | Reactivity of Antibodies to Each Serotype LPS | | | | | | | | | | | | | | |
| | [Serotype] | | | | | | | | | | | | | | |
| | A | B | C | D | E | F | G | H | I | J | K | L | M | Lipid A | Ra |
| HPs1 | - | - | - | + | - | - | - | - | + | - | - | - | - | - | - |
| HPs2 | - | - | - | - | + | + | - | - | - | - | - | - | - | - | - |
| Hps4 | + | - | - | - | - | - | - | - | - | - | - | + | - | - | - |
| HPs5 | - | - | - | - | - | - | + | + | - | - | - | - | - | - | - |
| HPs6 | - | - | - | - | + | + | - | - | - | - | - | - | - | - | - |
| HPs7 | + | - | - | - | - | + | - | - | - | - | - | - | - | - | - |
| HPs8 | - | - | - | - | + | + | - | - | - | - | - | - | - | - | - |
| HPs9 | - | - | - | + | - | - | - | - | + | - | - | - | - | - | - |
| HPs10 | - | - | - | - | + | + | - | - | - | - | - | - | - | - | - |
| HPs11 | - | - | - | - | - | - | + | + | - | - | - | - | - | - | - |
| HPs12 | + | - | - | - | - | + | - | - | - | - | - | - | - | - | - |

Example 11 Cross reactivity of human monoclonal antibodies with LPSs I

Cross reactivities of the human monoclonal antibodies HPs1 and HPs2 obtained in Example 1 with O-specific polysaccharide chain of Pseudomonas aeruginosa, namely, O-antigens were examined by the enzyme-linked immunosorbent assay (ELISA) using a plate coated with LPS. The Pseudomonas aeruginosa LPS prepared in Example 1 was dissolved in carbonate buffer (pH 9.6) to make a concentration of 2 $\mu$g/ml. In each well of a 96 well plate (Greiner) for ELISA, 50 $\mu$l of LPS solution was separately charged to the well, which was allowed to stand at 37°C for 2 hours. After fixing, the solution was discarded and blocked with 2% BSA-containing carbonate buffer. Each antibody (25 ng/ml) solution dissolved in PBS containing 0.05% Tween 20 was mixed with an equal amount of a 2-fold serial dilution of each Pseudomonas aeruginosa LPS solution (50 $\mu$g/ml). The mixture was reacted at room temperature for 1 hour. Fifteen microliters of the reaction solution was separately charged in each well of the above LPS-fixed 96 well plate for ELISA and reacted at room temperature for 1.5 hours. After washing the well with 0.05% Tween 20-containing PBS, a reaction was carried out with 50 $\mu$l of peroxidase-conjugated rabit anti-human immunoglobulin (500-fold dilution) at room temperature for 1.5 hours. After washing with 0.05% Tween 20-containing PBS, a reaction was carried out with 50 $\mu$l of citrate buffer containing 10 mg/ml of 2,2'-azinobis(3-ethylbenzthiazoline sulfonic acid) (ABTS) and 0.003% of hydrogen peroxide at room temperature for 30 minutes. Absorbancy at a wavelength of 414 nm was measured and, an inhibitory rate to binding of each antibody to LPS fixed onto the plate by each Pseudomonas aeruginosa LPS was determined from the absorbancy.

As shown in Figure 1, binding of HPs1 to the plate coated with LPS of group I Pseudomonas aeruginosa was inhibited by each of LPS of group I and group D Pseudomonas aeruginosa but not inhibited by LPS of group A Pseudomonas aeruginosa. Thus, it was confirmed that HPs1 was a monoclonal antibody of recongnizing two O-antigens of group I and group D in common. As shown in Figure 2, binding of HPs2 to the plate coated with LPS of group F Pseudomonas aeruginosa was inhibited by each LPS of group E

19

and group F Pseudomonas aeruginosa but not inhibited by LPS of group D Pseudomonas aeruginosa. Thus, it was confirmed that HPs2 was a monoclonal antibody of recognizing two O-antigens of group E and group F in common.

Example 12 Cross reactivity of human monoclonal antibodies with LPSs II

Cross reactivities of the human monoclonal antibodies HPs4, HPs5, HPs6, HPs7 and HPs8 obtained in Examples 2, 3, 4 and 5 with O-antigens of Pseudomonas aeruginosa were examined by ELISA using a plate coated with LPS in a manner similar to Example 11. As shown in Figure 3, binding of HPs4 to the plate coated with LPS of group A Pseudomonas aeruginosa was inhibited by each LPS of group A and group L Pseudomonas aeruginosa; as shown in Figure 4, binding of HPs5 to the plate coated with LPS of group H Pseudomonas aeruginosa was inhibited by each LPS of group G and group H Pseudomonas aeruginosa; as shown in Figure 5, binding of HPs6 to the plate coated with LPS of group E Pseudomonas aeruginosa was inhibited by each LPS of group E and group F Pseudomonas aeruginosa; as shown in Figure 6, binding of HPs7 to the plate coated with LPS of group A Pseudomonas aeruginosa was inhibited by each LPS of group A and group F Pseudomonas aeruginosa; and as shown in Figure 7, binding of HPs8 to the plate coated with LPS of group E Pseudomonas aeruginosa was inhibited by each LPS of group E and group F Pseudomonas aeruginosa. From these results, it was confirmed that HPs4, HPs5, Hps6, Hps7 and HPs8 were human monoclonal antibodies of recognizing two O-antigens of groups A and L, groups G and H, groups E and F, groups A and F, groups E and F, respectively, in common.

Example 13 Cross reactivity of human monoclonal antibodies with LPSs III

Cross reactivities of the human monoclonal antibodies HPs9, HPs10, HPs11 and HPs12 obtained in Examples 6, 7, 8 and 9 with O-antigens of Pseudomonas aeruginosa were examined by ELISA using a plate coated with LPS in a manner similar to Example 11. Binding of HPs9 to the plate coated with LPS of group I Pseudomonas aeruginosa was inhibited by each LPS of group D and group I Pseudomonas aeruginosa. Binding of HPs10 to the plate coated with LPS of group E Pseudomonas aeruginosa was inhibited by each LPS of group E and group F Pseudomonas aeruginosa. Binding of HPs11 to the plate coated with LPS of group H Pseudomonas aeruginosa was inhibited by each LPS of group G and group H Pseudomonas aerugionsa. Binding of HPs12 to the plate coated with LPS of group A Pseudomonas aeruginosa was inhibited by each LPS of group A and group F Pseudomonas aeruginosa. From these results, it was confirmed that HPs9, HPs10, HPs11 and HPs12 were human monoclonal antibodies of recognizing two O-antigens of groups D and I, groups E and F, groups G and H and, groups A and F, respectively, in common.

Example 14 Test for promoting effect of human monoclonal antibody on bactericidal activity of polymorphonuclear cells against Pseudomonas aeruginosa

Promoting effect of human monoclonal antibody HPs2 obtained in Example 1 on bactericidal activity of mouse polymorphonuclear cells against Pseudomonas aeruginosa was examined. PBS containing 2.5% (w/v) glycogen was intraperitoneally injected in 2 ml/mouse to mice (Balb/c, female) of 8 to 12 weeks old after birth. Five hours after, peritoneal exudate cells (collected from 14 mice) containing polymorphonuclear cells were gathered. The peritoneal exudate cells containing polymorphonuclear cells were washed twice with Hanks' solution under ice cooling and dispersed in 10 mM HEPES-containing Harks' balanced salt solution (hereafter simply referred to as HBSS) in a cell density of $1 \times 10^7$/ml, which was stored under ice cooling until it was provided for use. Group E Pseudomonas aeruginosa PA103 and group F Pseudomonas aeruginosa IID 1006 were inoculated on nutrient agar, respectively, followed by incubation at 37°C for 20 hours. Grown colonies were scraped out and suspended in physiological salt solution. After washing twice with the same solution, the bacterial cells were suspended in HBSS to become absorbancy of 0.12 at a wavelength of 540 nm. The prepared bacterial cell suspensions were stored under ice cooling until it was provided for use. As complement a 5-fold dilution of guinea pig normal serum (lyophilized complement, Nihon Biotest Laboratories) with HBSS was used.

Under ice cooling, 10 $\mu$l each of the bacterial cell suspension were separately charged in a plastic test tube (Falcon) and, 40 $\mu$l of HPs2 previously adjusted to concentrations of 25 $\mu$g/ml, 2.5 $\mu$g/ml and 0.25 $\mu$g/ml with HBSS and 10 $\mu$l of complement solution were added thereto. The mixture was thoroughly mixed. Next, 40 $\mu$l of peritoneal exudate cell suspension containing polymorphonuclear cells was added to the mixture. After thoroughly mixing, rotary shake culture (200 rpm) was carried out at 37°C for 2 hours. After completion of the incubation, 900 $\mu$l of ice cooled water was added to each test tube to osmotically puncture polymorphonuclear cells. 100 $\mu$l each was taken out of each test tube and diluted to 10-fold, 100-fold and 1000-fold with HBSS. Each dilution was inoculated on nutrient agar plate (3 dishes) by 100 $\mu$l each followed by incubation at 37°C for 18 hours. The number of colonies formed on the nutrient agar plate was counted to determine the remaining bacterial cell count. In the control group, HBSS was added instead of each addition solution. As shown in Tables 3 and 4, the results reveal that HPs2 promoted bactericidal activity of polymorphonuclear cells against different serotypes of group E and F Pseudomonas aeruginosa in the presence of complement.

Table 3

| Bactericidal Activity Against Group E Pseudomonas aeruginosa (PA103) | | | |
|---|---|---|---|
| Antibody ($\mu$g/ml) | Polymorphonuclear Cells | Complement | Count of Remained Viable Cells * (x $10^5$ CFU/ml) |
| 0 | - | - | 9.1 |
| 0 | + | + | 16.0 |
| 0.01 | + | + | 4.7 |
| 0.1 | + | + | 0.56 |
| 1 | + | + | 0.65 |
| 10 | + | + | 0.12 |

* When started = 6 x $10^5$ colony forming units (CFU)/ml

Table 4

| Bactericidal Activity Against Group F Pseudomonas Aeruginosa (IID 1006) | | | |
|---|---|---|---|
| Antibody ($\mu$g/ml) | Polymorphonuclear Cells | Complement | Count of Remained Viable Cells * (x $10^5$ CFU/ml) |
| 0 | - | - | 0.41 |
| 0.1 | + | + | 0.040 |
| 1 | + | + | 0.073 |
| 10 | + | + | 0.073 |

* When started = 2.5 x $10^5$ colony forming units (CFU)/ml

Example 15 Test on protective activity of human monoclonal antibody against Pseudomonas aeruginosa infection I

Protective activity of the human monoclonal antibodies HPs1, HPs2, HPs4, HPs5, HPs6, HPs7 and HPs8 obtained in Examples 1, 2, 3, 4 and 5 against Pseudomonas aeruginosa infection was examined. The human monoclonal antibody was intraperitoneally injected to mice (Balb/c, female) of 8 to 12 weeks old after birth, one group being 5 to 10 mice, in 0.2 ml of a solution containing 50 ng, 500 ng, 5 $\mu$g and 50 $\mu$g of each human monoclonal antibody per mouse. Two hours after, bacterial suspension of each strain (IID 1001 (group A), IID 1004 (group D), PA 103 (group E), F7 (group F), P 28 (group G), IID 1009 (group H), IID 1010 (group I), and IID 1014 (group L)) was intraperitoneally challenged. In the control group, physiological salt solution alone was injected instead of the human monoclonal antibody. Each serotype Pseudomonas aeruginosa was inoculated on heart infusion agar plate medium followed by incubation at 37°C overnight. Grown bacterial cell colonies were scraped out. After diluting with physiological salt solution, 5% mucin was added and adjusted to be a challenging bacterial amount of 3 to 15 times 50% lethal dose (LD$_{50}$ value) per mouse. After challenging Pseudomonas aeruginosa, 50% effective dose (ED$_{50}$ value) was determined based on survival rate of mice in each injection group on the 7th day. As shown in Table 5, the results reveal that each human monoclonal antibody had a high protective activity against infections with Pseudomonas aeruginosa of two different serotypes, each showing reaction specificity.

EP 0 327 648 B1

Table 5

| ED$_{50}$ Value ($\mu$g/mouse) | | | | | | | | |
|---|---|---|---|---|---|---|---|---|
| Monoclonal Antibody | [Serotype of Challenge Bacteria] | | | | | | | |
| | A | D | E | F | G | H | I | L |
| HPs1 | >50 | 0.5 | - | - | - | - | 5.0 | - |
| HPs2 | >50 | - | 0.055 | 0.1 | - | - | - | - |
| HPs4 | 0.1 | - | - | - | - | >50 | - | 0.1 |
| HPs5 | >50 | - | - | - | 0.08 | 0.1 | - | - |
| HPs6 | >50 | - | 0.08 | 0.1 | - | - | - | - |
| HPs7 | 0.5 | - | - | 0.5 | - | >50 | - | - |
| HPs8 | >50 | - | 0.1 | 0.1 | - | - | - | - |

Example 16 Test on protective activity of human monoclonal antibody against Pseudomonas aeruginosa infection II

Protective activity of the human monoclonal antibody HPs2 obtained in Example 1 against Pseudomonas aeruginosa infection was examined by varying time for injection of the antibody. The human monoclonal antibody HPs2 was intraperitoneally injected to mice (Balb/c, female) of 8 weeks old after birth, one group being 10 mice, in 0.2 ml of a solution containing 0.2 $\mu$g of HPs2 per mouse. The bacterial suspension of PA 103 (group E) was intraperitoneally challenged 24 hours and 2 hours prior to injection of the antibody, at the same time and 1 hour and 4 hours after. In the control group, physiological salt solution alone was injected instead of the human monoclonal antibody. The bacterial suspension was prepared in a manner similar to Example 15; i.e., after diluting with physiological salt solution, 5% mucin was added and adjusted to be a challenge bacterial amount of 8 times LD$_{50}$ per mouse. A survival rate of mice on the 3rd day after challenge of Pseudomonas aeruginosa was observed. As shown in Table 6, the results reveal that HPs2 showed protective activity against infection of group E Pseudomonas aeruginosa showing reaction specificity, except for injection four hours after challenge of the bacterial solution. From the results, it was made clear that HPs2 had not only prophylactic effect but also therapeutic effect against Pseudomonas aeruginosa infection.

Table 6

| Antibody ($\mu$g/mouse) | Time Injected | Surviving Number (3 days after) | Survival Rate (%) |
|---|---|---|---|
| 0 | simultaneous | 0/10 | 0 |
| 0.2 | before 24 hours | 6/10 | 60 |
| 0.2 | before 2 hours | 10/10 | 100 |
| 0.2 | simultaneous | 9/10 | 90 |
| 0.2 | 1 hour after | 6/10 | 60 |
| 0.2 | 4 hours after | 1/10 | 10 |

Example 17 Production of lyophilized preparation

HPs2 obtained in Example 1 was dissolved in PBS containing 0.2% (w/v) human serum albumin (Calbio) in a concentration of 1 mg/ml. The solution was filtered through membrane filter having a pore size of 0.22 microns for sterilization. The filtrate was aseptically charged in a vial by 1 ml each and lyophilized to produce a lyophilized preparation. The preparation was again dissolved in distilled water and titer to Pseudomonas aeruginosa was measured; its activity was maintained.

22

Effects of the Invention

By use of the human monoclonal antibodies according to the present invention singly or in combination of two or more or in combination with other human antibodies, excellent prophylactic and therapeutic effects against Pseudomonas aeruginosa infectious diseases can be achieved.

To be notable in the present invention is that the present inventors have found for the first time that antigens recognized by the single human monoclonal antibodies having cross reactivity between Pseudomonas aeruginosa of different serotypes are present on an O-specific polysaccharide chain which is a serotype specific antigenic site and the human monoclonal antibodies exhibit prophylactic and therapeutic effects against Pseudomonas aeruginosa infectious diseases in an extremely low concentration and low dose. The present inventors have further found that protective spectrum can be broadened by single use and such thus makes it possible to reduce the kind of human monoclonal antibodies to be mixed in production of preparations containing the human monoclonal antibodies to O-antigens of Pseudomonas aeruginosa.

In Published Unexamined Japanese Patent Application No. 155398/1986, human monoclonal antibody showing reactivity to bacteria of serotype 2, 7 and 13 according to the Homma's classification are described but all of the bacteria recognized by the monoclonal antibody are classified into the same serotype according to the serological classification of the Serotyping Committee for the Japan Pseudomonas aeruginosa Society and the Lanyi's classification. The human monoclonal antibody is considered to be an antibody merely recognizing substrain in the same serotype but is different in property from the single human monoclonal antibody which recognizes Pseudomonas aeruginosa of a plurality of different serotypes referred to in the present invention.

The term showing cross reactivity between serotypes of Pseudomonas aeruginosa as used in the present invention refers to a case in which a plurality of Pseudomonas aeruginosae with which a human monoclonal antibody shows reactivity are not classified into only one serotype in any of serological classifications hitherto known. In case that a plurality of Pseudomonas aeruginosa with which a human monoclonal antibody shows reactivity are classified into a plurality of serotypes only in a specific serological classification, such a case is not referred to as showing cross reactivity between serotypes of Pseudomonas aeruginosa but this shows that the human monoclonal antibody is reactive merely with substrains in the same serotype.

Furthermore in the human monoclonal antibodies of the present invention, type of immunoglobulin is IgM or IgG but may also be IgA, needless to say.

Microorganisms (desposited in Fermentation Research Institute), as used according to the invention are:

1. FERM P-9745
2. FERM P-9750
3. FERM P-9751
4. FERM P-9752
5. FERM P-9753
6. FERM BP-1596
7. FERM BP-1598
8. FERM BP-1599
9. FERM BP-1600

**Claims**

1. Human monoclonal antibody capable of recognizing simultaneously and reacting with each O-antigen of two serotypes of Pseudomonas aeruginosa, said two serotypes being groups A and F, groups A and L, groups D and I, and groups E and F, or groups G and H.

2. Human monoclonal antibody which is produced by FERM BP-1598, BP-1596, BP-1600, BP-1599, P-9750, P-9745, P-9751, P-9753, or P-9752.

3. Self-reproducing cell capable of producing a human monoclonal antibody as claimed in claims 1 and 2 and a cell line derived from said cell.

4. Self-reproducing cell as claimed in claim 3 and a cell line derived from said cell wherein said self-reproducing cell is an Epstein-Barr virus transformed cell.

5. Self-reproducing cell as claimed in claim 3 and a cell line derived from said cell wherein said self-reproducing cell is a hybridoma.

6. Hybridoma as claimed in claim 5 and a cell line derived from said hybridoma wherein said hybridoma is a hybridoma of a cell capable of indefinite growth and a human derived antibody producing cell.

7. Hybridoma as claimed in claim 6 and a cell line derived from said hybridoma wherein said cell capable of indefinite growth is a human derived cell.

8. Hybridoma as claimed in claim 6 and a cell line derived from said hybridoma wherein said cell capable of indefinite growth is an animal derived cell.

9. Hybridoma as claimed in claims 6 to 8 and a cell line derived from said hybridoma wherein said antibody producing cell is a normal lymphocyte.

10. Hybridoma as claimed in claims 6 to 8 and a cell line derived from said hybridoma wherein said antibody producing cell is a virus transformed cell.

11. Self-reproducing cell of FERM BP-1598 BP-1596, BP-1600, BP-1599, P-9750, P-9745, P-9751, P-9753, or P-9752, and a cell line derived therefrom.

12. Prophylactic and therapeutic composition against Pseudomonas aeruginosa infectious diseases comprising at least one human monoclonal antibody as claimed in claims 1 and 2.

13. Liquid preparation comprising at least one human monoclonal antibody as claimed in claims 1 and 2.

14. Lyophilized preparation comprising at least one human monoclonal antibody as claimed in claims 1 and 2.

**Patentansprüche**

1. Menschlicher monoklonaler Antikörper, der in der Lage ist, jeweils das O-Antigen zweier Serotypen von Pseudomonas aeruginosa zu erkennen und gleichzeitig damit zu reagieren, wobei die genannten zwei Serotypen die Gruppen A und F, A und L, D und I sowie E und F oder die Gruppen G und H darstellen.

2. Menschlicher monoklonaler Antikörper, der produziert wird von FERM BP-1598, BP-1596, BP-1600, BP-1599, P-9750, P-9745, P-9751, P-9753 oder P-9752.

3. Selbstreproduzierende Zelle, die in der Lage ist, einen menschlichen monoklonalen Antikörper nach den Ansprüchen 1 und 2 zu produzieren sowie eine Zellinie, die sich von der genannten Zelle ableitet.

4. Selbstreproduzierende Zelle nach Anspruch 3 und eine Zellinie, die sich von der genannten Zelle ableitet, worin die genannte selbstreproduzierende Zelle eine Epstein-Barr Virus-transformierte Zelle ist.

5. Selbsreproduzierende Zelle nach Anspruch 3 und eine Zellinie, die sich von der genannten Zelle ableitet, worin die selbstreproduzierende Zelle eine Hybridomzelle ist.

6. Hybridomzelle nach Anspruch 5 und eine Zellinie, die sich von der genannten Hybridomzelle ableitet, worin die genannte Hybridomzelle ein Hybridom einer Zelle ist, die die Fähigkeit zu unbegrenztem Wachstum besitzt, und eine Antikörper-produzierende Zelle, die sich vom Menschen ableitet.

7. Hybridom nach Anspruch 6 und eine von dem genannten Hybridom abgeleitete Zellinie, worin die genannte zu unbegrenztem Wachstum fähige Zelle eine Zelle ist, die sich vom Menschen ableitet.

8. Hybridom nach Anspruch 6 und eine Zellinie, die sich von dem genannten Hybridom ableitet, worin die genannte zu unbegrenztem Wachstum fähige Zelle eine Zelle ist, die sich vom Tier ableitet.

EP 0 327 648 B1

**9.** Hybridom nach den Ansprüchen 6 bis 8 und eine Zellinie, die sich von dem genannten Hybridom ableitet, worin die genannte Antiköper-produzierende Zelle ein normaler Lymphocyt ist.

**10.** Hybridom nach den Ansprüchen 6 bis 8 und eine Zellinie, die sich von dem genannten Hybridom ableitet, worin die genannte Antikörperproduzierende Zelle eine Virus-transformierte Zelle ist.

**11.** Selbstreproduzierende Zelle von FERM BP-1598, BP-1596, BP-1600, BP-1599, P-9750, P-9745, P-9751, P-9753 oder P-9752 und eine sich davon ableitende Zellinie.

**12.** Prophylaktisches und therapeutisches Präparat gegen Pseudomonas aeruginosa-Infektionskrankheiten, umfassend mindestens einen menschlichen monoklonalen Antikörper nach den Ansprüchen 1 und 2.

**13.** Flüssige Zubereitung, umfassend mindestens einen menschlichen monoklonalen Antikörper nach den Ansprüchen 1 und 2.

**14.** Lyophilisierte Zubereitung, umfassend mindestens einen menschlichen monoklonalen Antikörper nach den Ansprüchen 1 und 2.

**Revendications**

**1.** Anticorps monoclonal humain capable de reconnaître simultanément et de réagir avec chaque O-antigène de deux sérotypes de Pseudomonas aeruginosa, lesdits deux sérotypes étant les groupes A et F, les groupes A et L, les groupes D et I et les groupes E et F, ou les groupes G et H.

**2.** Anticorps monoclonal humain qui est produit par des organismes FERM BP-1598, BP-1596, BP-1600, BP-1599, P-9750, P-9745, P-9751, P-9753 ou P-9752.

**3.** Cellule autoreproductrice capable de produire un anticorps monoclonal humain suivant l'une quelconque des revendications 1 et 2 et une lignée de cellules dérivée de ladite cellule.

**4.** Cellule autoreproductrice suivant la revendication 3 et une lignée de cellules dérivée de ladite cellule, caractérisée en ce que ladite cellule autoreproductrice est une cellule de virus d'Epstein-Barr transformée.

**5.** Cellule autoreproductrice suivant la revendication 3 et une lignée de cellules dérivée de ladite cellule, caractérisée en ce que la cellule autoreproductrice est un hybridome.

**6.** Hybridome suivant la revendication 5 et une lignée de cellules dérivée dudit hybridome, caractérisé en ce que l'hybridome est un hybridome d'une cellule capable de croissance indéfinie et d'une cellule productrice d'anticorps dérivée de l'être humain.

**7.** Hybridome suivant la revendication 6 et une lignée de cellules dérivée dudit hybridome, caractérisé en ce que ladite cellule capable de croissance indéfinie est une cellule dérivée de l'être humain.

**8.** Hybridome suivant la revendication 6 et une lignée de cellules dérivée dudit hybridome, caractérisé en ce que ladite cellule capable de croissance indéfinie est une cellule dérivée d'un animal.

**9.** Hybridome suivant les revendications 6 à 8 et une lignée de cellules dérivée dudit hybridome, caractérisé en ce que ladite cellule productrice d'anticorps est un lymphocyte normal.

**10.** Hybridome suivant les revendications 6 à 8 et une lignée de cellules dérivée dudit hybridome, caractérisé en ce que ladite cellule productrice d'anticorps est une cellule de virus transformée.

**11.** Cellule autoreproductrice des organismes FERM BP-1598, BP-1596, BP-1600, BP-1599, P-9750, P-9745, P-9751, P-9753 ou P-9752 et une lignée de cellules qui en dérive.

**12.** Composition prophylactique et thérapeutique contre les maladies infectieuses à Pseudomonas aeruginosa, comprenant au moins un anticorps monoclonal humain tel que revendiqué dans les revendica-

25

tions 1 et 2.

**13.** Préparation liquide comprenant au moins un anticorps monoclonal humain tel que revendiqué dans les revendications 1 et 2.

**14.** Préparation lyophilisée comprenant au moins un anticorps monoclonal humain tel que revendiqué dans les revendications 1 et 2.

FIG. 1

FIG. 2

FIG. 3

FIG. 4

FIG. 5

FIG. 6

FIG. 7